Europäisches Patentamt

European Patent Office

Office européen des brevets

(11) Publication number: **0 001 924 B2**

(12) **NEW EUROPEAN PATENT SPECIFICATION**

(45) Date of publication of the new patent specification: **13.03.91 Bulletin 91/11**

(21) Application number: **78300587.9**

(22) Date of filing: **02.11.78**

(51) Int. Cl.⁵: **A61K 31/685, // A61K45/00**

(54) **Pharmaceutical composition for administering choline.**

The file contains technical information submitted after the application was filed and not included in this specification

(30) Priority: **02.11.77 US 847967**

(43) Date of publication of application: **16.05.79 Bulletin 79/10**

(45) Publication of the grant of the patent: **07.07.82 Bulletin 82/27**

(45) Mention of the opposition decision: **13.03.91 Bulletin 91/11**

(84) Designated Contracting States: **BE CH DE FR GB LU NL SE**

(56) References cited:
**CHEMICAL ABSTRACTS, vol. 81, 1974, Columbus, Ohio, USA, HAUBRICH D.R. et al. "Increase in tissue concentration of acetylcholine in guinea pigs in vivo induced by administration of choline", pages 921-7 abstract no. 45942x, 14(5)
CHEMICAL ABSTRACTS, Vol. 88, 1978, Columbus, Ohio, USA, R.J. WURTMAN et al. "Lecithin consumption raises serum-free-choline levels", page 224, left-hand column abstract no, 60447c, pages 68-9**

(73) Proprietor: **MASSACHUSETTS INSTITUTE OF TECHNOLOGY
77 Massachusetts Avenue
Cambridge, MA 02139 (US)**

(72) Inventor: **Growdon, John H.
21 Cedar Road
Brookline Massachusetts 02167 (US)**
Inventor: **Wurtman, Richard J.
271 Woodward Street
Waban Massachusetts 02168 (US)**

(74) Representative: **Barnard, Eric Edward
BROOKES & MARTIN High Holborn House
52/54 High Holborn
London WC1V 6SE (GB)**

EP 0 001 924 B2

## Description

Pharmaceutical composition for administering choline

This invention relates to the use of lecithin or lecithin analogs for the manufacture of medicaments. These compositions are provided for the administration of a drug with lecithin or lecithin analogs which dissociate to form choline in order to increase acethylcholine levels in brain and other tissues and alleviate human disorders arising as side-effects of the anti-psychotic drug as it is specified below in move detail.

There are a number of diseases which affect acetylcholine-containing neurons in the brain or other tissues, and which are treated by drugs that cause undesired side effects by diminishing acetylcholine release ; there also exist diseases now treated by other drugs in which the potency and/or efficiency of the drugs could be improved by combining them with choline or natural or synthetic compounds that dissociate to form choline in order thereby to enhance the release of acetylcholine. Such diseases include both those primarily involving the brain (e.g. diseases of higher cortical functions ; psychiatric illnesses ; movement disorders) and those involving the peripheral nervous system (e.g. neuromuscular disorders). Tardive dyskinesia is a particularly common movement disorder associated with inadequate release of brain acetylcholine as a result of drug administration for the initial brain disease (e.g. psychosis). Tardive dyskinesia is a choreic movement disorder characterized by involuntary twitches in the tongue, lips, jaw and extremities. It typically occurs in susceptible persons after chronic injestion of neuroleptic drugs and may involve an imbalance in the postulated reciprocal relation between dopaminergic and cholinergic neurons in the basal ganglions. Thus, drugs that either block catecholamine synthesis (e.g. alpha-methyl-p-tyrosine), deplete the brain of monoamines (e.g. reserpine, tetrabenazine) or antagonize dopamine's actions on synaptic receptors (e.g. pherothiazines, haloperidol) often suppress tardive dyskinesia, whereas drugs that indirectly stimulate dopamine receptors (e.g. amphetamine, levodopa) often exacerbate the abnormal movements. Drugs assumed to increase the amount of acetylcholine within brain synapses (e.g. physostigmine, deanol), also tend to suppress the chorea of tardive dyskinesia, whereas anticholinergics (e.g. scopolamine), make it worse.

It has been shown that choline administered by injection or by dietary supplementation increases blood choline levels in the rat ; (Cohen et al LIFE SCI., Vol. 16, 1095-1102, 1975 and SCIENCE Vol. 191, 561-562, 1976), this, in turn, increases choline levels in cholinergic neurons within the brain and elsewhere in the body, thereby accelerating the synthesis of acetylcholine, increasing tissue acetylcholine levels, and increasing the amounts of acetylcholine released into brain synapses. In human beings, oral doses of choline were found to cause dose-related increases in blood choline levels of sufficient magnitude (based on the studies on rats) to enhance brain acetylcholine synthesis and release ; choline levels in the cerebrospinal fluid also rose in parallel (Growdon et al., J. Neurochem Vol. 28, 229-231, 1977). It has also been reported (Davis et al. LIFE SCIENCES, Vol. 19, 1507-1516, 1976) in four human patients that the administration of choline decreased the choreiform movements of tardive dyskinesia ; no data were provided as to whether or not the drug given concurrently for psychosis (haloperidol, 3 mg per day) continued to be effective during the brief period of choline administration, and it was concluded that the apparent effectiveness of choline had to be interpreted with caution, since "... all four patients with tardive dyskinesia could have been gradually improving during the study" since this disease is characterized by extreme variability of clinical course. Thus, prior to our invention, it had not been known that the concomitant administration of lecithin along with an anti-psychotic drug that causes tardive dyskinesia as a side effect could significantly reduce or prevent the onset of tardive dyskinesia, without blocking the effectiveness of the drug in treating psychosis.

This invention is based upon the discovery that lecithin or a physiologically-acceptable lecithin analog that dissociates to form choline, when administered concomitantly with a drug, can, by increasing neuronal acetylcholine levels,

1) reduce or prevent undesirable side effects of the drug associated with inadequate acetylcholine release, and/or

2) potentiate the effectiveness of the drug. The lecithin and the drug may be administered orally such as in tablet, capsule or liquid form or parenterally by intravenous, intramuscular or subcutaneous injection. This invention is useful even with patients having a prior history of the undesirable side effect or of suboptimal therapeutic response or of therapeutic responses requiring a very large drug dose, but who continue taking the drug.

In accordance with this invention, lecithin (or a lecithin analog), which dissociates to form choline, is orally administered to a patient together with a drug in order to increase blood levels of choline, and thereby to increase the level of acetylcholine in the brain. The acetylcholine is synthesized from choline and acetyl CoA in a reaction catalyzed by choline acetyl-transferase (CAT). It has been found that the administration of lecithin to form choline potentiates the drug by reducing the incidence or suppressing side effects of the primary drug and/or that lower dosages of the primary drug are needed to attain the desired effects of the drug. While the results obtained will vary from patient to patient, the reduced side effects and increased efficacy observed are sufficiently significant as to justify the conclusion that their reduction is caused by administration of the compound that dissociates to form choline.

There are a number of brain and peripheral diseases involving cholinergic neurons that are presently treated with drugs that are only sometimes effective, or that require very large doses of the drugs (with correspondingly greater cost and incidence of side effects) ; some of these diseases can be more effectively treated by combining the existing drug therapy with concomitant lecithin or a lecithin analog that disscociates to form choline. One example is the mania phases of manic-depressive psychosis, which is currently treated with lithium salts. These salts, as a biochemical side effect, interfered with the uptake of choline into the brain ; this tends to reduce brain acetylcholine levels, which exacerbates the mania. The co-administration of a choline-producing compound with the lithium salts would allow more effective treatment of the mania, and a reduction in the lithium dose needed by most patients. Another example is myasthenia gravis, a peripheral disease involving the cholinergic nerves that innervate skeletal muscle. The current mode of treatment involves giving drugs like neostigmine (Prostigmin) that increase acetylcholine levels in neuromuscular synapses by blocking the degradation of this neurotransmitter. Were the compound that dissociates to form choline to be given concomitantly with the cholinesterase-inhibitor, the resulting increases in acetylcholine levels would both potentiate the effect of the cholinesterase-inhibitor and allow for a reduction in its dose.

Some of the drugs utilized in the present invention are those which cause significant undesirable effects. Representative of such drugs are neuroleptics, such as chlorpromazine (THORAZINE®) and haloperidol (HALDOL®) that are used in the treatment of such diseases as schizophrenia, Huntington's disease and Tourette's syndrome. Other drugs that cause undesired effects include phychomotor stimulants such as amphetamine (DEXADRINE®) and methyl-phenidate (RITALINE®) that are used to reat patients with minimal brain dysfunction, hyperactivity and specific dyslexias.

The effects of some other drugs utilized in this invention are potentiated. Representative of such drugs are : 1) isoxsuprine (VASODILAN®) and dihydroergotamines (HYDERGINE®) that are used in the treatment of senility ; 2) glucocorticosteroids such as triamcinotone (ARISTOCORT®) and prednisone (METICORTEN®) and anticholinesterase drugs such as neostigmine (PROSTIGMIN®) and pyridostigmine (MESTINON®) that are used to treat neuromuscular diseases, including polynigositis and myasthenia gravis ; 3) lithium (ESKALITH®) that is used to treat manic-depressive illness and 4) tranquillizers such as phenobarbital (LUMINAL®) and diazepam (VALIUM®) that are used to treat anxiety psychoneurosis.

Lecithin or lecithin analogs such as lyso-lecithin are used as the choline source since they are not degraded in the gut, in contrast to choline. They are administered so that a choline level of 20-30 nanomoles/ml and usually between at least 10 and 50 n moles/ml is attained in the patient's blood stream. When utilizing lecithin in a liquid carrier, such as a sweetened elixir, it is administered in amounts of between 0.1 and 50 g/day. When lecithin is administered in granular form as a tablet or in a capsule, it is employed in amounts of between 0.1 and 100 g/day, usually between 30 and 50 g/day. Normally, lecithin is not available as a pure compound and is available in admixture with other phospholipids wherein the lecithin comprises 20-30 weight percent of the mixture.

Using the composition as manufactured according to this invention, the compound that dissociates to choline is administered concomitantly with the drug. However the effect may be achieved when the compound is administered prior to the drug, but the period of time between the compound administration and the drug administration must be less than when acetylcholine concentration reduction begins to occur in the brain. Generally, the period of time between administrations is less than 36 hours, preferably less than 24 hours.

## Claims

1. The use of a drug and lecithin or a physiologically acceptable lecithin analog that dissociates to form choline to manufacture a medicament for concomitant use of the drug and lecithin or its analog in therapy wherein lecithin or its analog acts as an agent for increasing neuronal acetyl choline levels
   1) to alleviate undesired side-effects of the drug caused by inadequate acetyl choline release occasioned by use of the drug, or
   2) to potentiate the effectiveness of the drug in causing acetyl choline release, the lecithin or its analog being provided in an amount sufficient to cause a blood choline level of 10-50 nanomoles/ml.

2. The use of a drug and lecithin or a physiologically acceptable lecithin analog that dissociates to form choline to manufacture a medicament for sequential use of the drug and lecithin or its analog by administration of lecithin followed by the drug in therapy wherein lecithin or its analog acts as an agent for increasing neuronal acetyl choline levels
   1) to alleviate undesired side-effects of the drug caused by inadequate acetyl choline release occasioned by use of the drug, or

2) to potentiate the effectiveness of the drug in causing acetyl choline release, the lecithin or its analog being provided in an amount sufficient to cause a blood choline level of 10-50 nanomoles/ml.

3. The use of a drug and lecithin or a physiologically acceptable lecithin analog according to claim 1 that dissociates to form choline to manufacture a medicament for concomitant use of the drug and lecithin in therapy wherein lecithin or its analog acts as an agent for increasing neuronal acetyl choline levels

1) to alleviate undesired side-effects of the drug caused by inadequate acetyl choline release occasioned by use of the drug, or

2) to potentiate the effectiveness of the drug in causing acetyl choline release, the lecithin or its analog being provided in an amount sufficient to cause a blood choline level of from 20 to 30 nanomoles/ml.

4. The use of a drug and lecithin or a physiologically acceptable lecithin analog according to claim 2, that dissociates to form choline to manufacture a medicament for sequential use of the drug and lecithin or its analog by administration of lecithin followed by the drug in therapy wherein lecithin or its analog acts as an agent for increasing neuronal acetyl choline levels

1) to alleviate undesired side-effects of the drug caused by inadequate acetyl choline release occasioned by use of the drug, or

2) to potentiate the effectiveness of the drug in causing acetyl choline release, the lecithin or its analog being provided in an amount sufficient to cause a blood choline level of from 20-30 nanomoles/ml.

5. The use according to claim 1, 2, 3 and 4, wherein the drug is chlorpromazine, haloperidol or a lithium salt.

6. The use according to claim 1, 2, 3, and 4 wherein the drug is amphetamine, methyl phenidate, phenytoin, phenobarbital or diazepam.

7. The use according to claim 1, 2, 3 or 4, wherein the drug is a dihydroergotamine or a gluco-cortico steroid.

8. The use according to claim 1, 2, 3 or 4, wherein the drug is isoxsuprine, prednisone, neostigmine or pyridostigmine.

9. The use according to any one of claims 1 to 8 wherein the medicament is in capsule or tablet form.

10. The use according to any one of claims 1 to 8 wherein the medicament is in liquid form.

11. The use as claimed in any one of claims 1 to 8 wherein the medicament is formulated for oral administration.

**Revendications**

1. Utilisation d'une substance médicamenteuse et de lécithine, ou d'une lécithine physiologiquement acceptable analogue à celle qui se dissocie pour former de la choline, pour fabriquer un médicament pour un usage concomitant de la substance médicamenteuse et de la lécithine ou de son analogue dans la thérapie, dans laquelle la lécithine ou son analogue agit comme agent d'augmentation des valeurs d'acétyl choline neuronale,

1) pour diminuer les effets secondaires indésirables de la substance médicamenteuse causés par un dégagement inadéquat d'acétyl choline occasionné par l'usage de la substance médicamenteuse, ou

2) pour renforcer l'efficacité de la substance médicamenteuse en provoquant un dégagement d'acétyl choline, la lécithine ou son analogue étant fournis en quantité suffisante pour provoquer une teneur en choline dans le sang allant de 10 à 50 nanomoles/ml.

2. Utilisation d'une substance médicamenteuse et de lécithine, ou d'une lécithine physiologiquement acceptable analogue à celle qui se dissocie pour former de la choline, pour fabriquer un médicament pour un usage séquentiel de la substance médicamenteuse et de la lécithine ou de son analogue dans la thérapie, par administration de lécithine suivie de la substance médicamenteuse dans la thérapie, dans laquelle la lécithine ou son analogue agit comme agent d'augmentation des valeurs d'acétyl choline neuronale,

1) pour diminuer les effets secondaires indésirables de la substance médicamenteuse causés par un dégagement inadéquat d'acétyl choline occasionné par l'usage de la substance médicamenteuse, ou

2) pour renforcer l'efficacité de la substance médicamenteuse en provoquant un dégagement d'acétyle choline, la lécithine ou son analogue étant fournis en quantité suffisante pour provoquer une teneur en choline dans le sang allant de 10 à 50 nanomoles/ml.

3. Utilisation d'une substance médicamenteuse et de lécithine, ou d'une lécithine physiologiquement acceptable analogue à celle qui se dissocie pour former de la choline, selon la revendication 1, pour fabriquer un médicament pour un usage concomitant de la substance médicamenteuse et de la lécithine dans la thérapie, dans laquelle la lécithine ou son analogue agit comme agent d'augmentation des valeurs d'acétyl choline neuronale,

1) pour diminuer les effets secondaires indésirables de la substance médicamenteuse causés par un dégagement inadéquat d'acétyl choline occasionné par l'usage de la substance médicamenteuse, ou

2) pour renforcer l'efficacité de la substance médicamenteuse en provoquant un dégagement d'acétyl choline, la lécithine ou son analogue étant fournis en quantité suffisante pour provoquer une teneur en choline dans le sang allant de 20 à 30 nanomoles/ml.

4. Utilisation d'une substance médicamenteuse et de lécithine, ou d'une lécithine physiologiquement acceptable analogue à celle qui se dissocie pour former de la choline, selon la revendication 2, pour fabriquer un médicament pour un usage séquentiel de la substance médicamenteuse et de la lécithine ou de son analogue par administration de lécithine suivie de la substance médicamenteuse dans la thérapie, dans laquelle la lécithine ou son analogue agit comme agent d'augmentation des valeurs d'acétyl choline neuronale,

1) pour diminuer les effets secondaires indésirables de la substance médicamenteuse causés par un dégagement inadéquat d'acétyl choline occasionné par l'usage de la substance médicamenteuse, ou

2) pour renforcer l'efficacité de la substance médicamenteuse en provoquant un dégagement d'acétyl choline, la lécithine ou son analogue étant fournis en quantité suffisante pour provoquer une teneur en choline dans le sang allant de 20 à 30 nanomoles/ml.

5. Utilisation selon la revendication 1, 2, 3 ou 4, dans laquelle la substance médicamenteuse est le chlorpromazine, l'haloperidol ou un sel de lithium.

6. Utilisation selon la revendication 1, 2, 3 ou 4, dans laquelle la substance médicamenteuse est une amphétamine, le phénidate de méthyle, la phénytoine, le phénobarbitol ou le diazepam.

7. Utilisation selon la revendication 1, 2, 3 ou 4, dans laquelle la substance médicamenteuse est une dihydroergotamine ou un gluco-cortico stéroïde.

8. Utilisation selon la revendication 1, 2, 3 ou 4, dans laquelle la substance médicamenteuse est l'isoxsuprine, la prednisone, la neostigmine ou la pyridostigmine.

9. Utilisation selon l'une quelconque des revendications 1 à 8, dans laquelle le médicament se présente sous forme de capsules ou de tablettes.

10. Utilisation selon l'une quelconque des revendications 1 à 8, dans laquelle le médicament se présente sous forme liquide.

11. Utilisation selon l'une quelconque des revendications 1 à 8, dans laquelle le médicament est formulé pour administration orale.

**Ansprüche**

1. Verwendung eines pharmazeutischen Wirkstoffes und Lezithin oder ein physiologisch verträgliches, unter Bildung von Cholin dissoziierendes Lezithin-Analogon zur Herstellung eines Medikamentes für gleichzeitige Verabreichung des Wirkstoffes und Lezithin oder dessen Analogon bei der Therapie, wo Lezithin oder dessen Analogon als Mittel zur Erhöhung des neuronalen Acetylcholin-Spiegels wirkt, um

1) unerwünschte Nebenwirkungen des Wirkstoffes zu mildern, die durch unzureichende Acetylcholin-Freisetzung beim Gebrauch des Wirkstoffes verursacht werden, oder

2) die Wirkung des Wirkstoffes durch Freisetzung von Acetylcholin zu potenzieren, wobei das Lezithin oder dessen Analogon in einer für einen Blutcholinspiegel von 10-50 nano-Mole/ml ausreichenden Menge vorgesehen wird.

2. Verwendung eines pharmazeutischen Wirkstoffes und Lezithin oder ein physiologisch verträgliches, unter Bildung von Cholin dissoziierendes Lezithin-Analogon zur Herstellung eines Medikamentes für aufeinander folgende Verabreichung von Lezithin oder dessen Analogon und des Wirkstoffes, und zwar Verabreichung von Lezithin gefolgt von dem Wirkstoff, bei der Therapie, wo Lezithin oder dessen Analogon als Mittel zur Erhöhung des neuronalen Acetylcholin-Spiegels wirkt um

1) unerwünschte Nebenwirkungen des Wirkstoffes zu mildern, die durch unzureichende Acetylcholin-Freisetzung beim Gebrauch des Wirkstoffes verursacht werden, oder

2) die Wirkung des Wirkstoffes durch Freisetzung von Acetylcholin zu potenzieren, wobei das Lezithin oder dessen Analogon in einer für einen Blutcholinspiegel von 10-50 nano-Mole/ml ausreichenden Menge vorgesehen wird.

3. Verwendung eines pharmazeutischen Wirkstoffes und Lezithin oder ein physiologisch verträgliches, unter Bildung von Cholin dissoziierendes Lezithin-Analogon nach Anspruch 1 zur Herstellung eines Medikamentes für gleichzeitige Verabreichung des Wirkstoffes und Lezithin bei der Therapie, wo Lezithin oder dessen Analogon als Mittel zur Erhöhung des neuronalen Acetylcholin-Spiegels wirkt, um

1) unerwünschte Nebenwirkungen des Wirkstoffes zu mildern, die durch unzureichende Acetylcholin-Freisetzung beim Gebrauch des Wirkstoffes verursacht werden, oder

2) die Wirkung des Wirkstoffes durch Freisetzung von Acetylcholin zu potenzieren, wobei das Lezithin oder dessen Analogon in einer für einen Blutcholinspiegel von 20-30 nano-Mole/ml ausreichenden Menge vorgesehen wird.

4. Verwendung eines pharmazeutischen Wirkstoffes und Lezithin oder ein physiologisch verträgliches, unter Bildung von Cholin dissoziierendes Lezithin-Analogon nach Anspruch 2 zur Herstellung eines Medikamentes für aufeinander folgende Verabreichung von Lezithin oder dessen Analogon und des Wirkstoffes, und zwar Verabreichung von Lezithin gefolgt von dem Wirkstoff, bei der Therapie, wo Lezithin oder dessen Analogon als Mittel zur Erhöhung des neuronalen Acetylcholin-Spiegels wirkt, um

1) unerwünschte Nebenwirkungen des Wirkstoffes zu mildern, die durch unzureichende Acetylcholin-Freisetzung beim Gebrauch des Wirkstoffes verursacht werden, oder

2) die Wirkung des Wirkstoffes durch Freisetzung von Acetylcholin zu potenzieren, wobei das Lezithin oder dessen Analogon in einer für einen Blutcholinspiegel von 20-30 nano-Mole/ml ausreichenden Menge vorgesehen wird.

5. Verwendung gemäß einem der Ansprüche 1, 2, 3 oder 4, wobei als pharmazeutischer Wirkstoff Chlorpromazin, Haloperidol oder ein Lithiumsalz benutzt wird.

6. Verwendung gemäß einem der Ansprüche 1, 2, 3 oder 4, wobei als pharmazeutischer Wirkstoff Amphetamin, Methylphenidat, Phenytoin, Phenobarbitol oder Diazepam benutzt wird.

7. Verwendung gemäß einem der Ansprüche 1, 2, 3 oder 4, wobei als pharmazeutischer Wirkstoff ein Dihydroergotamin oder Glucocorticosteroid benutzt wird.

8. Verwendung gemäß einem der Ansprüche 1, 2, 3 oder 4, wobei als pharmazeutischer Wirkstoff Ixosuprin, Prednison, Neostigmin oder Pyridostigmin benutzt wird.

9. Verwendung gemäß irgend einem der Ansprüche 1 bis 8, wobei das Medikament in Form von Kapseln oder Tabletten formuliert wird.

10. Verwendung gemäß irgend einem der Ansprüche 1 bis 8, wobei das Medikament in flüssiger Form formuliert wird.

11. Verwendung gemäß irgend einem der Ansprüche 1 bis 8, wobei das Medikament in einer zur oralen Verabreichung geeigneten Form formuliert wird.